(19) Europäisches Patentamt / European Patent Office / Office européen des brevets

(11) **EP 4 252 791 B1**

(12) **EUROPEAN PATENT SPECIFICATION**

(45) Date of publication and mention
of the grant of the patent:
**25.03.2026 Bulletin 2026/13**

(21) Application number: **22887728.8**

(22) Date of filing: **31.10.2022**

(51) International Patent Classification (IPC):
*A61L 27/46* (2006.01)      *A61L 27/12* (2006.01)
*A61L 27/20* (2006.01)      *A61L 27/58* (2006.01)
*A61L 27/56* (2006.01)

(52) Cooperative Patent Classification (CPC):
(C-Sets available)
**A61L 27/56; A61L 27/46;** A61L 2400/06;
A61L 2430/34                                    (Cont.)

(86) International application number:
**PCT/KR2022/016783**

(87) International publication number:
**WO 2023/075534 (04.05.2023 Gazette 2023/18)**

(54) **COMPOSITE FILLER AND PRODUCT USING SAME**

ZUSAMMENGESETZTER FÜLLSTOFF UND PRODUKT DAMIT

CHARGE COMPOSITE ET PRODUIT L'UTILISANT

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **01.11.2021  KR 20210148252
28.10.2022  KR 20220141269**

(43) Date of publication of application:
**04.10.2023  Bulletin 2023/40**

(73) Proprietor: **LG Chem, Ltd.
Seoul 07336 (KR)**

(72) Inventors:
• **KIM, Yunseop
Daejeon 34122 (KR)**
• **KIM, Lucia
Daejeon 34122 (KR)**
• **KIM, Jee Seon
Daejeon 34122 (KR)**

(74) Representative: **Goddar, Heinz J.
Boehmert & Boehmert
Anwaltspartnerschaft mbB
Pettenkoferstrasse 22
80336 München (DE)**

(56) References cited:
JP-A- 2020 501 702      KR-A- 20190 118 694
KR-B1- 101 517 691      KR-B1- 102 181 033
US-A1- 2003 180 376      US-A1- 2006 093 670
US-A1- 2016 051 725      US-A1- 2017 112 969
US-A1- 2017 209 623

(52) Cooperative Patent Classification (CPC): (Cont.)

C-Sets
**A61L 27/46, C08L 5/08**

**Description**

**[TECHNICAL FIELD]**

The present disclosure relates to a composite filler having enhanced bioactive performance, and a product using the same.

**[BACKGROUND OF THE INVENTION]**

Fillers are complementary materials or contents that are injected or inserted into wrinkles, dented scars, or the like and are widely used in human organs such as wrinkles, scars, and vocal cords that require cosmetic surgery and volume retention.

Fillers are classified into permanent, semi-permanent, and temporary fillers in accordance with the retention period, and specific examples thereof include collagen, fat, hyaluronic acid, hydroxyapatite, polymethacryl, botox, and the like.

A conventionally used hyaluronic acid filler has the effect of imparting a voluminous feeling to the treated area, but has a drawback that it cannot fundamentally improve skin elasticity, has a too fast degradation rate and thus a short filler retention period.

In addition, calcium filler has the advantage that it promotes natural collagen production, exhibits a fundamental skin improvement effect, and has a slow degradation rate and thus a long retention period, but has the drawback that it is low in bioactive performance and slowly exhibits the skin improvement effect.

In order to overcome the limitations of such single component fillers, a method of using a composite filler that is a mixture of a biocompatible polymer such as carboxymethyl cellulose and calcium particles has been proposed. Composite fillers of carboxymethylcellulose and calcium particles overcome the limitations of single component fillers, and have the effect of retaining the initial volume and then stimulating the tissues with calcium particles to generate natural collagen, thus improving the skin itself.

However, the calcium particles applied to the conventionally used composite filler of carboxymethyl cellulose and calcium particles are spherical, high-density solid particles with a smooth surface, and have a limitation that their bioactive performance is low. In addition, there were difficulties in correction surgery due to the low biodegradability of carboxymethyl cellulose.

Therefore, there is a demand for the development of a filler that has improved bioactive performance compared to existing calcium particles, rapidly exhibits skin improvement effects, and enables initial volume retention and correction surgery.

US 2017/209623 A1 discloses a porous biocompatible polymer matrix having a plurality of macropores having an average size of about 100 μm to about 500 μm, and a calcium ceramic granule contacting the porous biocompatible matrix, the calcium ceramic granule having an average diameter of about 425 μm to about 800 μm and having an interconnected network of micropores defining at least one surface on an interior of the granule.

US 2017/112969 A1 discloses a hyaluronic acid/calcium phosphate composite prepared by sintering calcium phosphate powder to obtain a spherical calcium phosphate microsphere having a size of 45 μm to 75 μm; and mixing the calcium phosphate microsphere with a hyaluronic hydrogel cross-linked in the presence of a crosslinking agent.

US 2003/180376 A1 discloses a moldable putty composition comprising porous beta-TCP and a binder, wherein the binder is selected from the group consisting of sodium alginate, hyaluronic acid, sodium hyaluronate, gelatine, collagen, peptides, mucin, chondroitin sulfate, chitosan and others.

US 2006/093670 A1 discloses a composition comprising porous hydroxyapatite microparticles having pores charged by a biologically active drug, a human serum protein, and a mucopolysaccharide.

KR 20190118694 A discloses porous microsphere composites comprising porous microspheres and a polymer crosslinked within the pores of the microspheres.

[BRIEF SUMMARY OF THE INVENTION]

[Technical Problem]

The present disclosure is to provide a composite filler that introduces porous inorganic particles produced by a method with high production efficiency into a composite filler, enhances the bioactive performance of the composite filler, improves the skin improvement effect, and is highly biodegradable, thus enabling correction surgery.

The present disclosure is also to provide a product using a composite filler having enhanced bioactive performance.

**[Technical Solution]**

In order to achieve the above, provided is a composite filler comprising: porous inorganic particles including a sintered body of calcium-based particles and pores distributed in the sintered body, wherein the calcium-based particles include hydroxyapatite; and a biodegradable carrier, wherein the porous inorganic particles are contained in an amount of 1 part by weight to 50 parts by weight with respect to 100 parts by weight of the biodegradable carrier,

wherein the composite filler has a bioactivity degree according to the following Equation 1 of at least 25 mg/(kg·g):

Bioactivity degree = {[Calcium ion content in simulated body fluid (mg/kg)] - [Calcium ion content in simulated body fluid after immersing composite filler in simulated body fluid for 8 days (mg/kg)] }/(Inorganic particle content in composite filler (g)),     [Equation 1]

wherein a total pore volume of the porous inorganic particles is 0.01 $cm^3$/g to 0.05 $cm^3$/g,

wherein a specific surface area of the porous inorganic particles is 4 $m^2$/g to 10 $m^2$/g.

Also provided is a product comprising the above-mentioned composite filler.

Further embodiments are disclosed in the dependent claims.

**[DETAILED DESCRIPTION OF THE INVENTION]**

Hereinafter, a composite filler according to specific embodiments of the present disclosure and a product using the same will be described in more detail.

Unless otherwise specified throughout this specification, the technical terms used herein are only for reference to specific embodiments and is not intended to limit the present disclosure.

The singular forms "a", "an", and "the" used herein include plural references unless the context clearly dictates otherwise.

The term "including" or "comprising" as used herein specifies a specific feature, region, integer, step, action, element and/or component, but does not exclude the presence or addition of a different specific feature, region, integer, step, action, element, component and/or group.

Further, the terms including ordinal numbers such as "a first", "a second", etc. are used only for the purpose of distinguishing one component from another component, and are not limited by the ordinal numbers. For instance, a first component may be referred to as a second component, or similarly, the second component may be referred to as the first component, without departing from the scope of the present disclosure.

Now, the present disclosure will be described in more detail.

**1. Composite Filler**

According to one embodiment of the present disclosure, there is provided a composite filler comprising: porous inorganic particles including a sintered body of calcium-based particles and pores distributed in the sintered body, wherein the calcium-based particles include hydroxyapatite; and a biodegradable carrier, wherein the porous inorganic particles are contained in an amount of 1 part by weight to 50 parts by weight with respect to 100 parts by weight of the biodegradable carrier,

wherein the composite filler has a bioactivity degree according to the following Equation 1 of at least 25 mg/(kg·g):

Bioactivity degree = {[Calcium ion content in simulated body fluid (mg/kg)] - [Calcium ion content in simulated body fluid after immersing composite filler in simulated body fluid for 8 days (mg/kg)]}/(Inorganic particle content in composite filler (g)),     [Equation 1]

wherein a total pore volume of the porous inorganic particles is 0.01 $cm^3$/g to 0.05 $cm^3$/g,
wherein a specific surface area of the porous inorganic particles is 4 $m^2$/g to 10 $m^2$/g.

The present inventors have found through experiments that in the case of the composite filler of the one embodiment, porous inorganic particles including a sintered body of calcium-based particles and pores distributed in the sintered body are included together with a biodegradable carrier, whereby the stability of a biodegradable carrier and the bioactive performance of porous inorganic particles are combined to overcome the limitations of single component fillers, and have the effect of retaining the initial volume and then stimulating the tissues with calcium particles to generate natural collagen, thus improving the skin itself, and completed the present disclosure.

Particularly, the porous inorganic particles not only have a high porosity and specific surface area relative to conventional high-density solid particles and thus is improved in bioactive performance, and have the effect of rapidly inducing the surgery effect of fillers, but also have a fast particle production speed, and does not require an additional drying process and thus is highly productive, does not use organic solvents and thus is produced by a safe spray drying method without the risk of explosion, thereby maximizing the production efficiency.

Specifically, a composite filler of the one embodiment includes porous inorganic particles including a sintered body of calcium-based particles and pores distributed in the sintered body.

The calcium-based particles refer to particles containing a calcium element, and may be composed of only calcium elements or may be a mixture of calcium elements and other elements.

According to the invention, the calcium-based particles include hydroxyapatite. The hydroxyapatite is a calcium phosphate component which is an inorganic material constituting the teeth and bones of the human body, and has high biostability, promotes natural collagen production and is effective for fundamentally improving wrinkles.

The maximum diameter of the calcium-based particles may be 10 nm or more and 10 $\mu$m or less. The diameter of the calcium-based particles refers to the distance between two points where a straight line passing through the center of gravity of the particle meets the particle boundary line, and the largest value of these diameters corresponds to the maximum diameter. Examples of specific methods for measuring the maximum diameter of the calcium-based particles are not particularly limited, but can be confirmed, for example, through a TEM or SEM image.

When the maximum diameter of the calcium-based particles is excessively reduced to less than 10 nm, it is difficult to produce calcium-based secondary particles of several tens of $\mu$m or more through spray drying, and a high solids content is required to maintain a spherical shape. In addition, there may be a problem that the particle dispersibility in the slurry for spray drying is reduced. Further, when the maximum diameter of the calcium-based particles is excessively increased to more than 10 $\mu$m, there may be a problem that calcium-based secondary particles having low strength are produced.

The shape of the calcium-based particles is not particularly limited, and various shapes of conventionally known hydroxyapatite can be applied without limitation. An example of the shape of the calcium-based particles includes spherical, rod-like, acicular, linear, plate-like, sheet-like, and the like. Examples of specific methods for measuring the shape of the calcium-based particles are not particularly limited, but can be confirmed, for example, through a TEM or SEM image.

More specifically, the maximum diameter of the calcium-based particles is 10 nm or more and 200 nm or less, and the shape of the calcium-based particles may be acicular. The maximum diameter of the calcium-based particles is 10 nm or more and 200 nm or less, and when the shape of the calcium-based particles is spherical, the porous inorganic particles are not produced in a spherical form during the production thereof by the spray drying method, but are produced in an amorphous form, which may cause a problem that high injection power is required when injecting into the body.

In addition, the maximum diameter of the calcium-based particles may be 100 nm or more and 10 $\mu$m or less, or 1 $\mu$m or more and 10 $\mu$m or less, and the shape of the calcium-based particles may be spherical. The maximum diameter of the calcium-based particles is 100 nm or more and 10 $\mu$m or less, or 1 $\mu$m or more and 10 $\mu$m or less. If the shape of the calcium-based particles is not spherical, the surface roughness of the calcium-based secondary particles increases, which may cause a problem that a high injection force is required when injecting into the body.

Meanwhile, the porous inorganic particles includes a sintered body of the calcium-based particles. The sintered body of the calcium-based particles refers to a product obtained by subjecting for the aggregate of the plurality of calcium-based particles to a high-temperature sintering process. The sintering refers to a phenomenon in which, when powder, which is an aggregate of many particles, is heated to a temperature below the melting point, the powder melts and adheres to each other and solidifies. That is, the porous inorganic particles correspond to secondary particles obtained by sintering calcium-based powder in which a large number of the calcium-based primary particles are gathered.

Meanwhile, the porous inorganic particles include pores distributed in the sintered body. As the pores are distributed in the sintered body of the calcium-based particles, the porous inorganic particles may exhibit porosity. More specifically, the pores may be distributed inside and/or on the surface of the sintered body of calcium-based particles.

The pore refers to an empty space inside the sintered body of calcium-based particles, and may be used as meaning an opening, hollow, hole, void, or the like. As used herein, "porous particles" may refer to particles that have pores inside and/or on the surface of the particles.

As the porous inorganic particles contain pores distributed in the sintered body, the bioactive performance of the sintered body is improved due to an increase of the surface area by the pores, and a high skin improvement effect may appear rapidly.

The pores may be derived from the sintering process of the calcium-based particles, as described below.

Specifically, the pores correspond to spaces formed between the calcium-based particles by adjusting sintering conditions during sintering between the calcium-based particles.

That is, the porous inorganic particles may include a product obtained by the heat treatment of composite particles including a biocompatible binder and calcium-based particles. Through heat treatment of the composite particles including the biocompatible binder and calcium-based particles, the biocompatible binder is removed by thermal decomposition. As partial sintering between the calcium-based particles proceeds, fine pores may be introduced into the calcium-based particles. The details of the calcium-based particles can include all the contents described above for the composite filler.

The biocompatible binder can induce the calcium-based particles to be sufficiently aggregated in the composite particles in which the biocompatible binder is mixed with the calcium-based particles, thereby being useful for forming a sintered body.

The biocompatible binder may include at least one polymer selected from the group consisting of polyvinyl alcohol, polyvinylpyrrolidone, carboxymethylcellulose, and polyethylene glycol. That is, the biocompatible binder may include polymers that are polyvinyl alcohol, polyvinylpyrrolidone, carboxymethyl cellulose, polyethylene glycol, or mixtures of two or more thereof.

For a more specific example, the biocompatible binder may be polyvinyl alcohol. The polyvinyl alcohol has a boiling point of 228°C and can be removed while being vaporized at a temperature of 228°C or higher.

The weight average molecular weight of the biocompatible binder may be 100000 g/mol or more and 200000 g/mol or less, or 140000 g/mol or more and 190000 g/mol or less. As used herein, the weight average molecular weight refers to a polystyrene-converted weight average molecular weight measured by gel permeation chromatography(GPC). In the process of measuring the polystyrene-converted weight average molecular weight measured by GPC, a detector and an analytical column, such as a commonly known analysis apparatus and differential refractive index detector can be used, and commonly applied temperature conditions, solvent, and flow rate can be used. Specific examples of the measurement condition are as follows: Waters PL-GPC220 instrument was used and a Polymer Laboratories PLgel MIX-B 300 mm length column was used. An evaluation temperature was 160°C, and 1,2,4-trichlorobenzene was used for a solvent at a flow rate of 1 mL/min. Samples were prepared at a concentration of 10 mg/10 mL and then supplied in an amount of 200 μL, and the values of Mw could be determined using a calibration curve formed using a polystyrene standard. 9 Kinds of the polystyrene standards were used with the molecular weight of 2,000 / 10,000 / 30,000 / 70,000 / 200,000 / 700,000 / 2,000,000 / 4,000,000 / 10,000,000.

Thus, the heat treatment of the composite particles may include a primary heat treatment of the composite particles at a temperature of 450°C or more and 550°C or less, and a secondary heat treatment at a temperature of 600°C or more and 1200°C or less. In the process of the primary heat-treatment of the composite particles at a temperature of 450°C or more and 550°C or less, or 480°C or more and 520°C or less, the biocompatible binder may be vaporized and removed by thermal decomposition.

More specifically, the primary heat treatment may be performed by heating to a temperature of 450°C to 550°C or 480°C to 520°C at a temperature rising rate of 2°C/minute or more and 8°C/minute or less, or 4°C/minute or more and 6°C/minute or less, and then heat-treating at a temperature of 450°C to 550°C or 480°C to 520°C for 1 hour or more to 3 hours or less.

Further, in the process of secondary heat treatment of the composite particles at a temperature of 600°C to 1200°C, or 600°C to 1000°C, while the composite particles including pores and calcium-based particles are sintered, the porous inorganic particles including the above-mentioned sintered body of calcium-based particles, and pores distributed in the sintered body can be formed.

If the sintering temperature is excessively decreased to less than 600°C in the secondary heat treatment process, it is difficult to secure sufficient strength of the sintered body, and if the sintering temperature is excessively increased to more than 1200°C, the pores can be completely removed to form non-porous inorganic particles.

More specifically, the secondary heat treatment is performed by heating to a temperature of 600°C or more and 1200°C or less at a temperature rising rate of 2°C/min or more and 8°C/min or less, or 4°C/min or more and 6°C/min or less, and then heat-treating at a temperature of 600°C or more and 1200°C or less for 1 hour or more and 3 hours or less.

Meanwhile, the content of the calcium-based particles may be 5 parts by weight or more and 100 parts by weight or less, or 10 parts by weight or more and 90 parts by weight or less, or 12 parts by weight or more and 80 parts by weight or less, or 5 parts by weight or more and 30 parts by weight or less, or 40 parts by weight or more and 100 parts by weight or less with respect to 1 part by weight of the biocompatible binder.

When the content of the calcium-based particles is excessively increased with respect to 1 part by weight of the biocompatible binder, due to the relative decrease in the weight of the binder, it is difficult for the composite particles to form spheres during spray drying. On the other hand, when the content of the calcium-based particles is excessively reduced with respect to 1 part by weight of the biocompatible binder, due to the relative decrease in the weight of the calcium-based particles, the strength of the composite particles after sintering is low, which makes it difficult to maintain the shape, and the shape of the particles may be distorted.

More specifically, the content of the calcium-based particles may be 5 parts by weight or more and 30 parts by weight or less with respect to 1 part by weight of the biocompatible binder, and the shape of the calcium-based particles may be acicular. At this time, the maximum diameter of the acicular calcium-based particles may be 10 nm or more and 200 nm or less.

Further. the content of the calcium-based particles may be 40 parts by weight or more and 100 parts by weight or less with respect to 1 part by weight of the biocompatible binder, and the shape of the calcium-based particles may be spherical. At this time, the maximum diameter of the spherical calcium-based particles may be 100 nm or more and 10 $\mu$m or less, or 1 $\mu$m or more and 10 $\mu$m or less.

Meanwhile, the composite particles including the biocompatible binder and calcium-based particles may be a spray-dried product of a composition containing a biocompatible binder and calcium-based particles. The spray-dried product refers to a product obtained by spray-drying a composition containing a biocompatible binder and calcium-based particles. The spray drying has a fast particle production speed, does not require an additional drying process, has high productivity, and does not use an organic solvent, and thus is produced through a safe spray drying method without explosion-proof risk, which thus maximizes production efficiency.

Thus, it can have an advantage in significant effects compared to the emulsion method, which is another production method that can be used for particle formation. In the case of the emulsion method, it is limited in that oil or an organic solvent must be further used for forming the emulsion, and the washing and drying steps must be accompanied, which leads to the decrease in productivity.

The shape of the composite particles is not particularly limited, but may be spherical as an example. Examples of specific methods for measuring the shape of the composite particles is not particularly limited, but can be confirmed, for example, through an SEM image.

The average value of the maximum diameter of the composite particles may be 1 $\mu$m or more and 100 $\mu$m or less. The diameter of the composite particle refers to the distance between two points where a straight line passing through the center of gravity of the particle meets the particle boundary line, and the largest value of these diameters corresponds to the maximum diameter. Further, the maximum diameter is measured for each of the plurality of composite particles, and the value obtained by arithmetic average of these is called the average value of the maximum diameter. Examples of specific methods for measuring the maximum diameter of the composite particles are not particularly limited, but can be confirmed, for example, through an SEM image.

The composite particles may be a group of individual particles having an average value of maximum diameter of 1 $\mu$m or more and 100 $\mu$m or less, and individual microparticles included in such groups may have an average maximum diameter of 1 $\mu$m or more and 100 $\mu$m or less. More specifically, 95% or 99% of the individual microparticles included in the above group may have a maximum diameter of 1 $\mu$m or more and 100 $\mu$m or less.

As the porous inorganic particles contained in the composite filler of one embodiment include a sintered body of calcium-based particles and pores distributed in the sintered body as described above, the bioactive performance is improved, and the skin improvement effect appears rapidly.

The shape of the porous inorganic particles is not particularly limited, but an example may be spherical. Examples of specific methods for measuring the shape of the porous inorganic particles are not particularly limited, but can be confirmed, for example, through an SEM image. As the shape of the porous inorganic particles satisfies a spherical shape, it can be injected into the body with a low injection force and can induce a low immune response in the body.

The specific surface area of the porous inorganic particles is 4 m$^2$/g to 10 m$^2$/g, or 6 m$^2$/g to 10 m$^2$/g. The specific surface area is measured using a BET analyzer. As the specific surface area of the porous inorganic particles satisfies the above range, the porosity and specific surface area are higher than those of conventional high-density solid particles, so that the bioactivity is improved and the effect of quickly inducing the treatment effect of the filler can be realized. Meanwhile, when the specific surface area of the porous inorganic particles is excessively reduced to 0.1 m$^2$/g or less, the porosity and specific surface area are low similar to the conventional high-density solid particles, and the bioactive performance is reduced while the surgery effect of the filler is reduced, which may cause a problem that a large number of particles is necessary. Further, when the specific surface area of the porous inorganic particles is excessively increased, the strength of the sintered body of inorganic particles is lowered, which may pose a problem that the usable process is limited during production of the composite filler.

Further, the total pore volume of the porous inorganic particles is 0.01 cm$^3$/g to 0.05 cm$^3$/g, or 0.016 cm$^3$/g to 0.05 cm$^3$/g, or 0.017 cm$^3$/g to 0.05 cm$^3$/g. The total pore volume refers to the total volume of all pores contained in the porous inorganic particles, and is measured using a BET analyzer. As the total pore volume of the porous inorganic particles satisfies the above range, the porosity and specific surface area are higher than those of conventional high-density solid particles, so that the bioactivity is improved and the effect of quickly inducing the treatment effect of the filler can be realized. Meanwhile, when the total pore volume of the porous inorganic particles is excessively decreased to 0.001 m$^2$/g or less or the like, the porosity and specific surface area are low similar to the conventional high-density solid particles, and the bioactive performance is reduced while the surgery effect of the filler is reduced, which may cause a problem that many particles is necessary. In addition, when the total pore volume of the porous inorganic particles is excessively increased, the strength of the sintered body of inorganic particles is lowered, which may pose a problem that the usable process is limited during production of the composite filler.

Further, the average value of the maximum diameter of the porous inorganic particles may be 1 μm to 1000 μm, or 10 μm to 100 μm, or 10 μm to 45 μm. The diameter of the porous inorganic particle refers to the distance between two points where a straight line passing through the center of gravity of the particle meets the particle boundary line, and the largest value of these diameters corresponds to the maximum diameter. Further, the maximum diameter is measured for each of the plurality of porous inorganic particles, and the value obtained by arithmetic average of these is called the average value of the maximum diameter. Examples of specific methods for measuring the maximum diameter of the composite particles are not particularly limited, but can be confirmed, for example, through an SEM image.

The porous inorganic particles may be a group of individual particles having an average value of maximum diameter of 1 μm to 1000 μm, or 10 μm to 100 μm, or 10 μm to 45 μm, and individual microparticles included in such groups may have an average maximum diameter of 1 μm to 1000 μm, or 10 μm to 100 μm, or 10 μm to 45 μm. More specifically, 95% or 99% of the individual microparticles included in the above group may have a maximum diameter of 1 μm to 1000 μm, or 10 μm to 100 μm, or 10 μm to 45 μm.

As the average value of the maximum diameter of the porous inorganic particles satisfies the above range, it is possible to maximize the skin improvement effect without the side effects in the body or the pain of the surgery. When the average value of the maximum diameter of the porous inorganic particles is excessively reduced to less than 1 μm, there may be a problem that an excessive foreign matter reaction in the body may occur. On the other hand, when the average value of the maximum diameter of the porous inorganic particles is excessively increased to more than 1000 μm, or the like, there may be a problem that the specific surface area per particle mass decreases, which not only reduces the effect of the surgery, but also induces great pain during the surgery.

Meanwhile, the composite filler includes a biodegradable carrier. The biodegradable carrier acts as a substrate, matrix or carrier of the composite filler, and the porous inorganic particles can be dispersed inside or outside the biodegradable carrier, as described below. When the porous inorganic particles are dispersed inside or outside the biodegradable carrier, the porous inorganic particles can be evenly dispersed while the biodegradable carrier and the porous inorganic particles are in direct contact with each other. The biodegradable carrier and the porous inorganic particles may be evenly distributed through physical dispersion without chemically bonding.

That is, the biodegradable carrier can come into contact with the surface of the porous inorganic particles. No chemical bond exists between the porous inorganic particles and the biodegradable carrier. When another coating layer (e.g., silane coating layer) is formed on the surface of the porous inorganic particles and the coating layer comes into contact with the biodegradable carrier to form a chemical bond, it is difficult to sufficiently realize the effect of improving the bioactive performance of composite fillers by porous inorganic particles, which may cause a problem that not only the efficiency of the process, which requires an excessive amount of porous inorganic particles to increase bioactivity, is reduced and the cost is increased, but also a high injection force is required when a filler mixed with an excessive amount of porous inorganic particles is injected into the body.

Examples of the biodegradable carrier are not particularly limited, and various biodegradable carriers widely used in the filler field are applicable without limitation. In one example, the biodegradable carrier may include gelatin, hyaluronic acid (HA), carboxymethyl cellulose (CMC), chondroitin (sulphate), dextran (sulphate), chitosan, collagen, carboxymethyl chitin, fibrin, pullulan, polylactide, polyglycolide (PGA), polylactide-glycolide copolymer (PLGA), polyanhydride, polyorthoester, polyetherester, polycaprolactone, polyethylene glycol (PEG), cyclodextrin, Poloxamer, or a mixture of two or more thereof, etc.

Preferably, hyaluronic acid may be included as the biodegradable carrier. The hyaluronic acid is a biosynthetic natural material abundantly present in the skin of animals and the like, and is a hydrophilic material due to its large number of hydroxyl groups (-OH), and acts as a moisturizing agent in the skin of animals and the like. It reacts with the CD44 protein expressed in various epithelial cells and regulates various physiological actions.

The composite filler includes 1 to 50 parts by weight, or 1 to 30 parts by weight, or 1 to 10 parts by weight of the porous inorganic particles with respect to 100 parts by weight of the biodegradable carrier. When the content of the porous inorganic particles is excessively reduced with respect to 100 parts by weight of the biodegradable carrier, the skin improvement effect due to bioactive performance may not appear. In addition, when the content of the porous inorganic particles is excessively increased with respect to 100 parts by weight of the biodegradable carrier, porous inorganic particles in the composite filler may be unevenly dispersed, which may cause difficulties in smoothly injecting into the body.

The composite filler has a bioactivity degree according to the following Equation 1 of 25 mg/(kg·g) or more, or 30 mg/(kg·g) or more, or 30.5 mg/(kg·g) or more, or 35 mg/(kg·g) or more, or 40 mg/(kg·g) or more, or 46 mg/(kg·g) or more, or 100 mg/(kg·g) or more, or 25 mg/(kg·g) to 100 mg/(kg·g), or 30 mg/(kg·g) to 100 mg/(kg·g), or 30.5 mg/(kg·g) to 100 mg/(kg·g), or 35 mg/(kg·g) to 100 mg/(kg·g), or 40 mg/(kg·g) to 100 mg/(kg·g), or 46 mg/(kg·g) to 100 mg/(kg·g).

Bioactivity degree = {[Calcium ion content in simulated body fluid (mg/kg)] - [Calcium ion content in simulated body fluid after immersing composite filler in simulated body fluid for 8 days (mg/kg)] }/(Inorganic particle content in composite filler (g) ).  [Equation 1]

Also, the composite filler may have a bioactivity degree according to the following Equation 2 of 15 mg/(kg·g) or more, or 20 mg/(kg·g) or more, or 22 mg/(kg·g) or more, or 25 mg/(kg·g) or more, or 32 mg/(kg·g) or more, or 35 mg/(kg·g) or more, or 100 mg/(kg·g) or less, or 15 mg/(kg·g) to 100 mg/(kg·g), or 20 mg/(kg·g) to 100 mg/(kg·g), or 22 mg/(kg·g) to 100 mg/(kg·g), or 25 mg/(kg·g) to 100 mg/(kg·g), or 32 mg/(kg·g) to 100 mg/(kg·g) , or 35 mg/(kg·g) to 100 mg/(kg·g).

Bioactivity degree = {[Phosphorus ion content in simulated body fluid (mg/kg)] - [Phosphorus ion content in simulated body fluid after immersing composite filler in simulated body fluid for 8 days (mg/kg)] }/(Inorganic particle content in composite filler (g) ).  [Equation 2]

As the bioactivity degree of the composite filler according to Equation 1 or Equation 2 satisfies the above range, the bioactivity is improved and the skin improvement effect appears rapidly, and the initial volume retention and correction surgery are possible.

On the other hand, if the bioactivity degree according to Equation 1 or 2 is excessively reduced, there is a drawback that the bioactive performance is low, and the skin improvement effect appears slowly.

Meanwhile, the composite filler may further include various additive components commonly included in fillers, for example, a lubricant such as glycerin, a phosphate buffer, and the like, as needed.

## 2. Products

According to yet another embodiment of the present disclosure, there can be provided a product comprising the composite filler of the one embodiment. The details of the composite filler include all the contents described above in one embodiment.

Examples of the above products are not particularly limited and can be applied without restriction depending on the application for which the filler is applied. Examples of the above products include foods, pharmaceuticals, cosmetics, and the like.

## [Advantageous Effects]

According to the present disclosure, a composite filler that introduces porous inorganic particles produced by a method with high production efficiency into the composite filler, enhances the bioactivity of the composite filler to improve the skin improvement effect, and is highly biodegradable and thus can be used for correction surgery, and a product using the same.

## [BRIEF DESCRIPTION OF THE DRAWINGS]

Fig. 1 shows surface and cross-sectional SEM images of the porous inorganic particles obtained in Example 1;
Fig. 2 shows a SEM image of the surface of the porous inorganic particles obtained in Example 2;
Fig. 3 shows surface and cross-sectional SEM images of the porous inorganic particles obtained in Example 3;
Fig. 4 shows surface and cross-sectional SEM images of the porous inorganic particles obtained in Example 4;
Fig. 5 shows a SEM image of the surface of the porous inorganic particles obtained in Example 5; and
Fig. 6 shows surface and cross-sectional SEM images of the inorganic particles obtained in Comparative Example 1.

The present disclosure will be described in more detail with reference to examples. However, the following examples are for illustrative purposes only, and the scope of the present disclosure is not intended to be limited thereby.

## <Example: Production of composite filler with improved bioactivity>

Example 1

(1) Production of porous inorganic particles

Polyvinyl alcohol (PVA, weight average molecular weight 146,000 ~ 186,000 Da, 99+% hydrolyzed) was stirred in water at 90°C to prepare a 1 wt.% PVA aqueous solution.

Acicular hydroxyapatite (HAp) powder having a maximum diameter of 150 nm was added to the PVA aqueous solution so that the weight ratio of HAp/PVA satisfies 12/1 to prepare a suspension.

The suspension was spray-dried (Buchi mini spray dryer B-290), and when drying was completed, particles were obtained and put in a crucible and kept at 500°C for 2 hours in a box furnace to remove PVA, which was then sintered again at 1000 °C for 2 hours to produce porous inorganic particles.

(2) Production of composite filler

0.4 g of the porous inorganic particles were mixed with 9.6 g of hyaluronic acid to produce a composite filler.

Example 2

Porous inorganic particles and composite fillers were produced in the same manner as in Example 1, except that the maximum diameter of hydroxyapatite (HAp) powder was changed to 10 nm to 50 nm as shown in Table 1 below.

Example 3

(1) Production of porous inorganic particles

Polyvinyl alcohol (PVA, weight average molecular weight 146,000 ~ 186,000 Da, 99+% hydrolyzed) was stirred in water at 90°C to prepare a 1 wt.% PVA aqueous solution.

Spherical hydroxyapatite (HAp) powder having a maximum diameter of 2.5 μm was added to the PVA aqueous solution so that the weight ratio of HAp/PVA satisfies 50/1 to prepare a suspension.

The suspension was spray-dried (Buchi mini spray dryer B-290), and when drying was completed, particles were obtained and put in a crucible and kept at 500°C for 2 hours in a box furnace to remove PVA, which was then sintered again at 1000°C for 2 hours to produce porous inorganic particles.

(2) Production of composite filler

0.4 g of the porous inorganic particles were mixed with 9.6 g of hyaluronic acid to produce a composite filler.

Example 4

Porous inorganic particles and composite fillers were produced in the same manner as in Example 3, except that the sintering temperature was changed to 1200°C as shown in Table 1 below.

Example 5

Porous inorganic particles and composite fillers were produced in the same manner as in Example 3, except that a suspension was prepared so that the weight ratio of HAp/PVA satisfies 80/1 as shown in Table 1 below.

**<Comparative Example: Production of Composite Filler>**

Comparative Example 1

Inorganic particles and composite fillers were produced in the same manner as in Example 1, except that the sintering temperature was changed to 1200°C as shown in Table 1 below.

**<Reference Example: Production of Composite Filler>**

Reference Example 1

93.25% by volume of methanol was mixed with 3.93% by volume of distilled water. 0.81% by volume of acetic acid was added to buffer the solution to pH 4.5~5.5. 2% by volume of 3-glycidoxypropyltrimethoxysilane was added to the solution to prepare a 3-glycidoxypropyltrimethoxysilane solution.

0.4 g of the porous inorganic particles obtained in (1) of Example 1 was added to the 3-glycidoxypropyltrimethoxysilane solution for 30 minutes, and then cured at 70°C for 24 hours to introduce a silane layer.

The porous inorganic particles into which the silane layer was introduced were mixed with 9.6 g of hyaluronic acid to produce a composite filler in which a chemical bond between the silane layer and hyaluronic acid was formed.

### <Experimental Example>

The physical properties of the inorganic particles and composite fillers obtained in Examples, Comparative Example, or Reference Example were measured by the following method, and the results are shown in tables and figures.

### 1. Particle shape

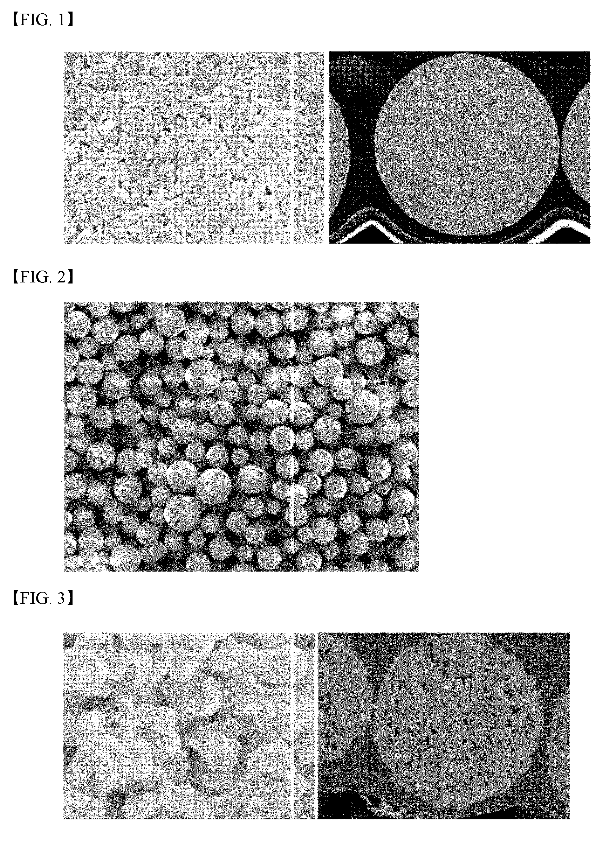

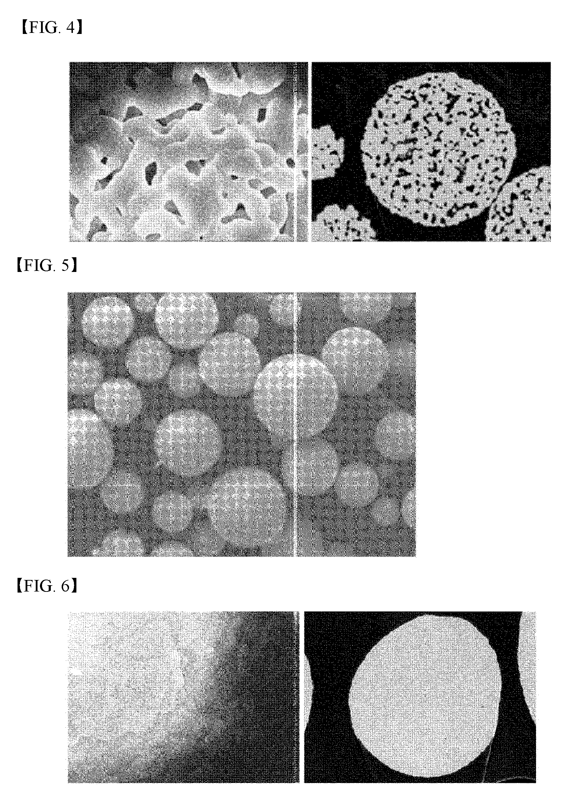

For the inorganic particles obtained in Examples and Comparative Example, the shape of the surface or cross section was confirmed through the SEM image, and shown in Figs. 1 to 6, respectively.

### 2. Particle size

For the inorganic particles obtained in Examples and Comparative Examples, the maximum diameter of each 100 particle was measured through an SEM image, and the arithmetic mean of these values was obtained.

### 3. Porosity

For the inorganic particles obtained in Examples and Comparative Examples, the cross-sectional shape of the particles was confirmed through the SEM image, and the porosity was expressed according to the presence or absence of pores as follows.

○: presence of pores on the cross-sectional SEM image of the inside of the particle
X: No presence of pores on the cross-sectional SEM image of the inside of the particle

### 4. Specific surface area and total pore volume

For the inorganic particles obtained in Examples and Comparative Example, the specific surface area and total pore volume were measured using a BET analyzer.

### 5. Bioactivity degree

The composite fillers obtained in Examples, Comparative Example and Reference Example were immersed in a simulated body fluid for 8 days, samples were prepared by an acid digestion method, and ICP-OES equipment was used for the samples. The contents of Ca ions and P ions (unit: mg/kg) were measured, and the bioactivity degrees according to Equation 1 and Equation 2 below were evaluated, respectively. The content of Ca ions and P ions in the simulated body fluid was determined to be 42 mg/kg, which means that the larger the value of the following Equation, the better the bioactivity degree.

Bioactivity degree = {[Calcium ion content in simulated body fluid (mg/kg)] - [Calcium ion content in simulated body fluid after immersing composite filler in simulated body fluid for 8 days (mg/kg)] }/(Inorganic particle content in composite filler (g) ).          [Equation 1]

Bioactivity degree = {[Phosphorus ion content in simulated body fluid (mg/kg)] - [Phosphorus ion content in simulated body fluid after immersing composite filler in simulated body fluid for 8 days (mg/kg)] }/(Inorganic particle content in composite filler (g)).          [Equation 2]

[Table 1]

| Experimental Example Measurement Results of Examples and Comparative Examples | | | | | | | | | |
|---|---|---|---|---|---|---|---|---|---|
| Category | Weight ratio of HAp/PVA | Maximum diameter of HAp | Shape of HAp | Sintering temperature | Particle shape | Particle size ($\mu$m) | Porosity | Specific surface area ($m^2/g$) | Total pore volume ($cm^3/g$) |
| Example 1 | 12/1 | 150 nm | Acicular | 1000 °C | Fig. 1 | 44 | ○ | 6 | 0.0171 |
| Example 2 | 12/1 | 10~50 nm | Acicular | 1000 °C | Fig. 2 | 43 | ○ | 6 | 0.0169 |
| Example 3 | 50/1 | 2.5 $\mu$m | Spherical | 1000 °C | Fig. 3 | 42 | ○ | 5 | 0.0146 |
| Example 4 | 50/1 | 2.5 $\mu$m | Spherical | 1200 °C | Fig. 4 | 34 | ○ | 4 | 0.0123 |
| Example 5 | 80/1 | 2.5 $\mu$m | Spherical | 1000 °C | Fig. 5 | 44 | ○ | 5 | 0.0151 |
| Comparative Example 1 | 12/1 | 150 nm | Acicular | 1200 °C | Fig. 6 | 49 | X | 0.1 | 0.0009 |

As shown in Table 1, it can be confirmed that in the case of inorganic particles contained in the composite fillers of Examples, porous inorganic particles having pores inside the particles were obtained, and both the specific surface area and pore volume were significantly improved as compared to Comparative Example 1. On the other hand, it can be confirmed that in the case of inorganic particles contained in the composite filler of Comparative Example 1, non-porous inorganic particles with no pores inside the particles were obtained, and both the specific surface area and pore volume were significantly reduced compared to Examples.

[Table 2]

| Bioactivity degree measurement results of Examples and Comparative Examples | | |
|---|---|---|
| Category | Ca ion bioactivity degree (mg/(kg·g)) | P ion bioactivity degree (mg/(kg·g)) |
| Example 1 | 47.2 | 36.1 |
| Example 2 | 45.7 | 33.2 |
| Example 3 | 30.6 | 23.6 |
| Example 4 | 30.2 | 21.9 |
| Example 5 | 37.3 | 30.5 |
| Comparative Example 1 | 20.8 | 12.5 |
| Reference Example 1 | 22.7 | 13.3 |

As shown in Table 2, it can be confirmed that in the case of the composite fillers of Examples, the Ca ion bioactivity degree was 30.2 mg/(kg g) to 47.2 mg/(kg g) and the P ion bioactivity degree was 21.9 mg/(kg g) to 36.1 mg/(kg g), which was larger than in Comparative Example, thus being excellent in bioactive performance. On the other hand, it can be confirmed that in the case of the composite filler of Comparative Example, the Ca ion bioactivity degree was 20.8 mg/(kg g) and the P ion bioactivity degree was 12.5 mg/(kg g), which was less than in Examples, thus exhibiting low bioactivity and poor skin improvement effect.

In addition, it can be confirmed that in the case of the composite filler of Reference Example, the Ca ion bioactivity degree was 22.7 mg/(kg g) and the P ion bioactivity degree was 13.3 mg/(kg g), which was less than in Examples, thus exhibiting low bioactivity and poor skin improvement effect.

**Claims**

1.    A composite filler comprising:

porous inorganic particles including a sintered body of calcium-based particles and pores distributed in the sintered body, wherein the calcium-based particles include hydroxyapatite; and
a biodegradable carrier,
wherein the porous inorganic particles are contained in an amount of 1 part by weight to 50 parts by weight with respect to 100 parts by weight of the biodegradable carrier,
wherein the composite filler has a bioactivity degree according to the following Equation 1 of at least 25 mg/(kg·g):

Bioactivity degree = {[Calcium ion content in simulated body fluid (mg/kg)] - [Calcium ion content in simulated body fluid after immersing composite filler in simulated body fluid for 8 days (mg/kg)]}/(Inorganic particle content in composite filler (g)),    [Equation 1]

wherein a total pore volume of the porous inorganic particles is 0.01 $cm^3$/g to 0.05 $cm^3$/g,
wherein the total pore volume is measured as disclosed in the specification,
wherein a specific surface area of the porous inorganic particles is 4 $m^2$/g to 10 $m^2$/g,
wherein the specific surface area is measured as disclosed in the specification.

2. The composite filler according to claim 1, wherein:
the composite filler has a bioactivity degree according to the following Equation 2 of at least 15 mg/(kg·g):

Bioactivity degree = {[Phosphorus ion content in simulated body fluid (mg/kg)] - [Phosphorus ion content in simulated body fluid after immersing composite filler in simulated body fluid for 8 days (mg/kg)]}/(Inorganic particle content in composite filler (g) ).    [Equation 2]

3. The composite filler according to claim 1, wherein:
the porous inorganic particles are dispersed inside or outside the biodegradable carrier.

4. The composite filler according to claim 1, wherein:
the biodegradable carrier is in contact with a surface of the porous inorganic particles.

5. The composite filler according to claim 1, wherein:
the biodegradable carrier includes hyaluronic acid.

6. A product comprising the composite filler as set forth in claim 1.


**Patentansprüche**

1. Verbundfüllstoff, umfassend:

poröse anorganische Teilchen, die einen Sinterkörper aus Teilchen auf Calciumbasis und Poren, die in dem Sinterkörper verteilt sind, einschließen, wobei die Teilchen auf Calciumbasis Hydroxyapatit einschließen; und einen biologisch abbaubaren Träger,
wobei die porösen anorganischen Teilchen in einer Menge von 1 Gewichtsteil bis 50 Gewichtsteilen, bezogen auf 100 Gewichtsteile des biologisch abbaubaren Trägers, enthalten sind,
wobei der Verbundfüllstoff einen Bioaktivitätsgrad gemäß der folgenden Gleichung 1 von mindestens 25 mg/(kg·g) aufweist:

Bioaktivitätsgrad = {[Calciumionengehalt in simulierter Körperflüssigkeit (mg/kg)] - [Calciumionengehalt in simulierter Körperflüssigkeit nach Eintauchen des Verbundfüllstoffs in simulierte Körperflüssigkeit für 8 Tage (mg/kg)]}/(Gehalt an anorganischen Teilchen in Verbundfüllstoff (g)),    [Gleichung 1]

wobei ein Gesamtporenvolumen der porösen anorganischen Teilchen 0,01 $cm^3$/g bis 0,05 $cm^3$/g beträgt,
wobei das Gesamtporenvolumen wie in der Beschreibung offenbart gemessen wird,
wobei eine spezifische Oberfläche der porösen anorganischen Teilchen 4 $m^2$/g bis 10 $m^2$/g beträgt,
wobei die spezifische Oberfläche wie in der Beschreibung offenbart gemessen wird.

**2.** Verbundfüllstoff nach Anspruch 1, wobei:
der Verbundfüllstoff einen Bioaktivitätsgrad gemäß der folgenden Gleichung 2 von mindestens 15 mg/(kg·g) aufweist:

Bioaktivitätsgrad = {[Phosphorionengehalt in simulierter Körperflüssigkeit (mg/kg)] - [Phosphorionengehalt in simulierter Körperflüssigkeit nach Eintauchen des Verbundfüllstoffs in simulierte Körperflüssigkeit für 8 Tage (mg/kg)]}/(Gehalt an anorganischen Teilchen in Verbundfüllstoff (g)).  [Gleichung 2]

**3.** Verbundfüllstoff nach Anspruch 1, wobei:
die porösen anorganischen Teilchen innerhalb oder außerhalb des biologisch abbaubaren Trägers dispergiert sind.

**4.** Verbundfüllstoff nach Anspruch 1, wobei:
der biologisch abbaubare Träger mit einer Oberfläche der porösen anorganischen Teilchen in Kontakt steht.

**5.** Verbundfüllstoff nach Anspruch 1, wobei:
der biologisch abbaubare Träger Hyaluronsäure einschließt.

**6.** Produkt, umfassend den Verbundfüllstoff nach Anspruch 1.

**Revendications**

**1.** Charge composite comprenant :

des particules inorganiques poreuses incluant un corps fritté constitué de particules à base de calcium et de pores distribués dans le corps fritté, où les particules à base de calcium incluent l'hydroxyapatite ; et
un véhicule biodégradable,
où les particules inorganiques poreuses sont contenues à une quantité qui va de 1 partie en poids à 50 parties en poids pour 100 parties en poids du véhicule biodégradable,
où la charge composite présente un niveau de bioactivité, déterminé conformément à l'Équation 1 ci-dessous, d'au moins 25 mg/(kg·g) :

Niveau de bioactivité = {[Teneur en ions calcium dans un liquide corporel simulé (mg/kg)] - [Teneur en ions calcium dans le liquide corporel simulé après immersion de la charge composite dans le liquide corporel simulé pendant 8 jours (mg/kg)]}/[Teneur en particules inorganiques dans la charge composite (g)],  [Équation 1]

où un volume total des pores des particules inorganiques poreuses va de 0,01 cm$^3$/g à 0,05 cm$^3$/g,
où le volume total des pores est mesuré comme divulgué dans la spécification,
où une superficie spécifique des particules inorganiques poreuses va de 4 m$^2$/g à 10 m$^2$/g,
où la superficie spécifique est mesurée comme divulgué dans la spécification.

**2.** Charge composite selon la revendication 1, où :
la charge composite présente un niveau de bioactivité, déterminé conformément à l'Équation 2 ci-dessous, d'au moins 15 mg/(kg·g) :

Niveau de bioactivité = {[Teneur en ions phosphore dans un liquide corporel simulé (mg/kg)] - [Teneur en ions phosphore dans le liquide corporel simulé après immersion de la charge composite dans le liquide corporel simulé pendant 8 jours (mg/kg)]}/[Teneur en particules inorganiques dans la charge composite (g)].  [Équation 2]

**3.** Charge composite selon la revendication 1, où :
les particules inorganiques poreuses sont dispersées dans le véhicule biodégradable ou hors de celui-ci.

**4.** Charge composite selon la revendication 1, où :

le véhicule biodégradable est en contact avec une surface des particules inorganiques poreuses.

5. Charge composite selon la revendication 1, où :
   le véhicule biodégradable inclut l'acide hyaluronique.

6. Produit comprenant la charge composite décrite à la revendication 1.

【FIG. 1】

【FIG. 2】

【FIG. 3】

【FIG. 4】

【FIG. 5】

【FIG. 6】

**EP 4 252 791 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2017209623 A1
- US 2017112969 A1
- US 2003180376 A1
- US 2006093670 A1
- KR 20190118694 A